# EUROPEAN PATENT APPLICATION

(11) **EP 1 867 727 A1**
(43) Date of publication of application: **19.12.2007**
(21) Application number: 06388043.9
(22) Date of filing: 15.06.2006
(51) Int. Cl.: C12P 7/48

(54) **Enhanced citrate production**

(71) Applicant: Danmarks Tekniske Universitet, 2800 Kgs. Lyngby (DK)
(72) Inventor: Nielsen, Jens, 2920 Charlottenlund (DK); de Jongh, Wian, 2000 Frederiksberg (DK)
(74) Representative: Klinge, Ulla Callesen

(57) **Abstract**

The present invention discloses a method for enhanced fungal production of citrate by the cytosolic over-production of malate and/or fumarate and/or succinate.

## Description

The present invention relates to methods for enhanced microbial production of citrate.

More particularly the invention relates to methods for improved production of citrate in industrially relevant filamentous fungi, such as *Aspergillus niger.*

The invention further relates to methods for enhanced microbial production of citrate, wherein the production media is not treated in order to remove Mn²⁺.

Citrate is the most intensively produced fungal bulk chemical product with a global market of more than 900 000 tons/ year (2001). *Aspergillus niger* fermentations meets the biggest part of this demand. Despite decades of study, only a small part of the production process is understood in details (Karaffa L. and Kubicek C.P., Appl.Microbiol.Biotechnol., 61, 189, 2003), but it has long been suspected that the principal mode of citrate export from the mitochondria involves the anti-port of cytosolic malate by the mitochondrial citrate transporter (CTP) (Ruijter G.J.G., Kubicek C.P., and Visser J., Production of Organic Acids by Fungi, in The Mycota X. Industrial Applications., Osiewacz, Eds., Springer-Verlag, Berlin, Heidelberg, 2002, 213). No detailed study has so far been reported on the affinity of CTP to different dicarboxylic acids in filamentous fungi such as *A. niger.* The tight regulation of the central carbon metabolism of A. *niger* has, however, been amply demonstrated by the lack of success in directly increasing citrate productivity through the over-expression of central carbon metabolism genes thus far. Torres and co-workers used biological systems theory coupled with constrained linear optimization to show that at least 7 glycolytic enzymes needed to be over-expressed to achieve a significant increase in flux towards citrate (Torres N.V., Voit E., and Gonzalez-Alcon C., Biotechnol Bioeng, 49, 247, 1996). Further, disappointing results have been obtained by the single (citrate synthase (Ruijter, Panneman, Xu, and Visser, FEMS Microbiol.Lett., 184, 35, 1-3-2000)) and double (pyruvate kinase and phosphofructokinase (Ruijter, Panneman, and Visser, Bio-chim.Biophys.Acta, 1334, 317, 15-3-1997)) over-expression of genes involved in citrate biosynthesis from glucose. The only successful attempts to enhance citrate production in filamentous fungi have been through indirect means: the disruption of the trehalose-6-phosphate synthase A (T6PSA)-encoding gene (ggsA), which leads to decreased inhibition of hexokinase by trehalose-6-phosphate (Arisan-Atac, Wolschek, and Kubicek, FEMS Microbiol.Lett., 140, 77, 15-6-1996); and anti-sense expression of *Brsa-25* (a possible amino acid transporter), which allows production in the presence of possible amino acid transporter), which allows production in the presence of normally inhibiting Mn²⁺ concentrations.

Accordingly, there is an ubiquitous need in the art for improving microbial production of citrate in industrially relevant microorganisms, and it is evident that genetic over-expression of genes involved in the central carbon metabolism has until now not led to significant improvements in this respect.

### Definitons:

By the phrase "genes involved in the production of malate and/or fumarate and/or succinate" is meant genes that, when transcribed and translated, result in proteins or peptide gene products that directly provide production of fumarate on/or succinate using other carbon sources as substrate. Examples of such genes are genes encoding malate dehydrogenases, fumerases and fumarate reductases. The specific examples employed during the experimental study leading to the invention are the genes *MDH2, Fum1, FumR* and *Frds1.*

By the term "gene products" is generally meant both mRNA produced by transcription of the relevant genes as well as peptides or proteins resulting from the translation of the produced mRNA's. Preferably, however, the term "gene products" refers to peptides or proteins. More preferably the term refers to enzymes.

By the term "over-expression" is meant the over-production of the relevant gene products facilitated by genetic engineering compared with the production of that product in the parent strain. Over-expression may thus be obtained simply by gene insertions but may alternatively also be achieved using other methods, such as increased transcription and/or translation, as well as decreased degradation or export of the relevant gene products from the relevant cell compartment. Methods for obtaining "over-expression" are generally well-known in the art and comprise conventional methods such as geneinsertions, improving gene promoter- and enhancer-sequences, improving codon usage, truncating or adding tail sequences encoding re-localisation or degradation or resistance to degradation of the gene product. By the term "cytosolic over-expression" is meant over-expression in the cell cytosol.

According to the invention it has surprisingly been shown that enhanced microbial production of citrate may be achieved by cytosolic over-expression of genes involved in the production of malate and/or fumarate and/or succinate.

In a particular preferred embodiment it has been shown that enhanced microbial production of citrate may be achieved by cytosolic over-expression of genes involved in the production of fumarate and/or succinate.

According to one embodiment it has additionally been shown that enhanced microbial production of citrate may be achieved by cytosolic localisation of gene products involved in the production of malate and/or fumarate and/or succinate.

In a particularly preferred embodiment it has been shown that enhanced microbial production of citrate may be achieved by cytosolic localisation of gene products from genes involved in the production of fumarate and succinate.

In one embodiment the genes involved in the production of malate and/or fumarate and/or succinate are chosen among the group comprising malate dehydrogenases, malate synthases, fumerate reductases, fumarate hydratases and fumerases.

In a particular embodiment the genes involved in the production of malate and/or fumarate and/or succinate are chosen among the group comprising malate dehydrogenases, fumerate reductases and fumerases.

In a particularly preferred embodiment the genes involved in the production of fumarate and/or succinate are chosen among the group comprising fumerate reductases and fumerases.

The microorganism according to the invention is a fungal organism capable of producing citrate. Preferably, the capability of producing citrate is of natural origin, however, this capability may as well be a result of genetic engineering. More generally, the micro-organism may be a species belonging to the genus *Aspergillus, e.g. A. terreus, A. oryzae, A. awamori, A. nidulans,* a species belonging to the genus *Penicillium,* a species belonging to the genus *Candida, e.g. C. utilis C. tropicalis, C. lipolytica,* a species belonging to the genus *Saccharomyces,* e.g. *S. cerevisiae, S. kluyveri,* or other fungal species, e.g. *Debaromyces hansenii, Hansenula polymorpha, Yarrowia lipolytica, Zygosaccharomyces rouxii* or *Schizosaccharomyces pombe, Rhizopus oryzae*.

In one embodiment the micro-organism according to the invention is a filamentous fungus. In a preferred embodiment of the invention the citrate producing micro-organism is a strain of *Aspergillus niger.*

It has further been demonstrated that enhanced microbial production of citrate achieved by cytosolic over-expression of genes involved in the production of malate and/or fumarate and/or succinate according to the invention lead to the loss of intolerance to Mn²⁺. The presence of Mn²⁺ is normally problematic for industrial producer strains, and therefore problematic in relation to methods of microbial production of citrate.

The invention is explained in greater detail below with reference to the accompanying drawings.

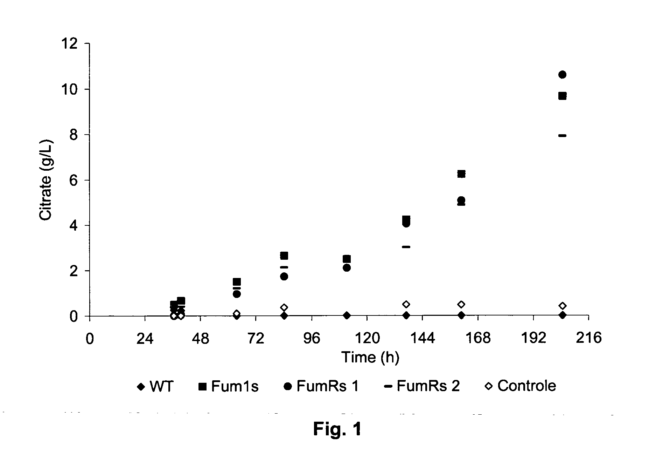

Fig. 1 shows the effect of cytosolic fumarase over-expression on citrate production over time. Fig. 1 also shows the production of citrate (in g/Litres) over fermentation time (0-200 hours) measured for different strains of A. *niger.* (WT) designates wild type, (Fum1s) designates a strain over-producing a cytosolicly targeted fumerase *(fum1* from S. *cerevisae),* (FumRs 1) designates a strain over-producing a cytosolicly targeted fumerase *(fumR* from R. *oryzae),* (FumRs 2) is a second *fumRs* overexpressing strain, (control) designates wildtype transformed with plasmid without fumerase gene.

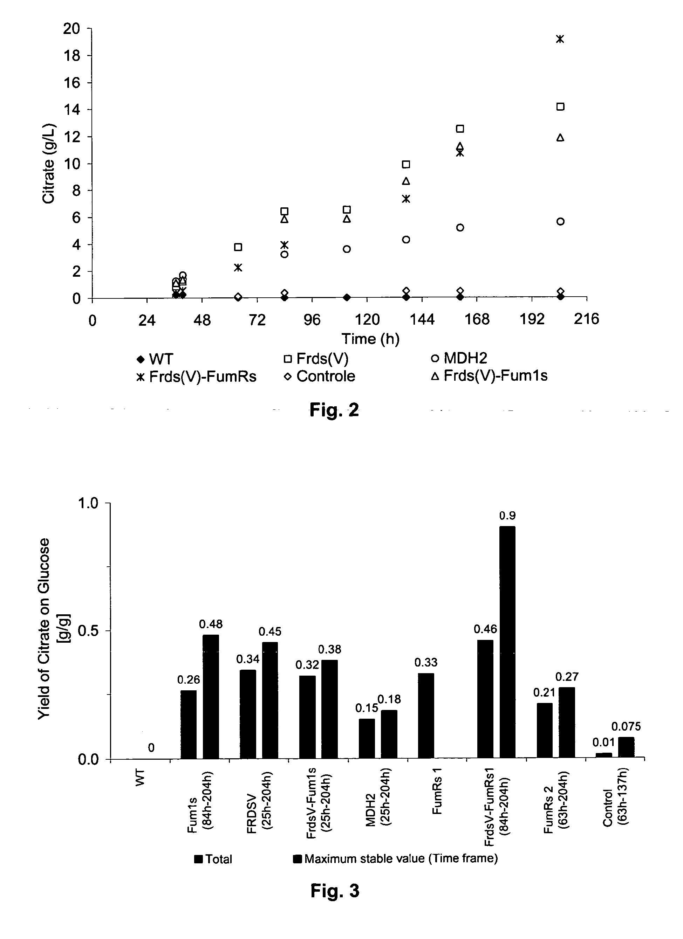

Fig. 2 shows the effect of over-expression (by insertion) of *frds1* and *Mdh2,* as well as the effect of the secondary insertion of *fumRs* or *fum1s* into the *frds1* containing Frds(V) strain. Fig. 2 also shows the production of citrate (in g/Litres) over fermentation time (0-200 hours) measured for different strains of A. *niger.* (WT) designates wild type, (Frds(V)) designates strain over-producing fumarate reductase, (MDH2) designates strain over-producing malate dehydrogenase, (Frds(V)-FumRs) designates strain over-producing fumarate reductase and fumRs-fumerase, (Frds(V)-Fum1s) designates strain over-producing fumarate reductase and fum1s-fumerase, (control) designates wildtype transformed with plasmid without fumerase gene.

Fig. 3 shows the citrate yield of strains overproducing malate dehydrogenase, fumarase and fumarate reductase according to the invention. The citrate yield of all the tested strains are given as *g citrate produced*/ *g glucose consumed.* The total citrate yield represents the final citrate concentration divided by the consumed glucose. The maximum stable value represents the maximum sustained yield achieved during the fermentation, and the time frame in which it was achieved is given between brackets. The total fermentation time would be 0h-204h. As the yield of citrate on glucose continued to increase through out the fermentation for FumRs 1, no maximum stable yield could be determined.

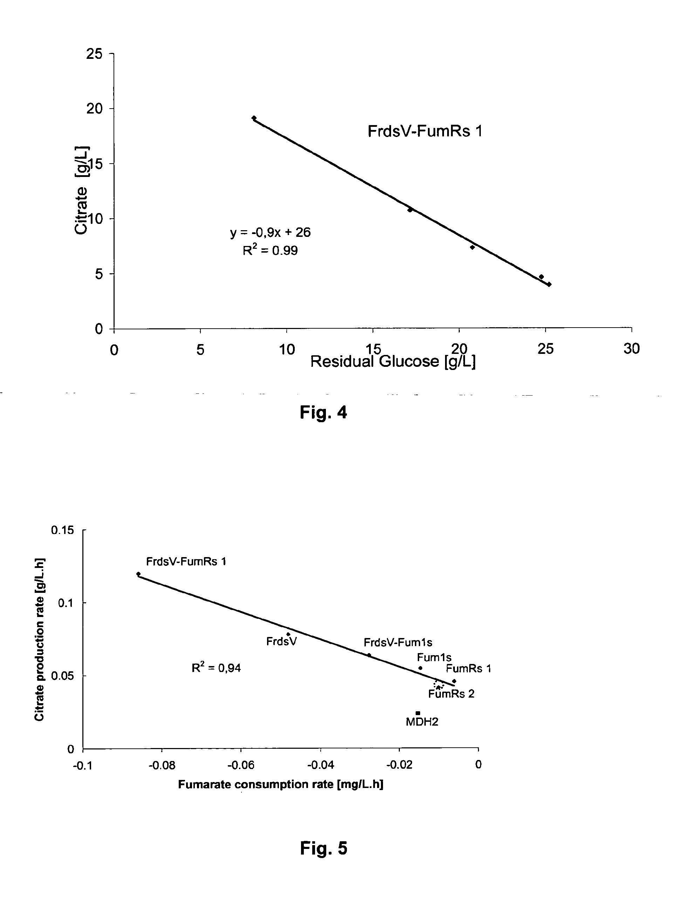

Fig. 4 illustrates the citrate concentration versus residual glucose concentration in the time frame 84h to 204h for the Frds(V)-FumRs 1 (strain over-producing fumarate reductase and fumRs-fumerase), i.e. the data used to calculate the maximum stable value of the yield.

Fig. 5 shows the citrate production rate versus fumarate consumption rate in the later stages of fermentation. (Fum1s) designates strain over-producing fum1s-fumerase, (FumRs 1) designates strain over-producing fumRs 1-fumerase, (FumRs 2) designates - strain over-producing fumRs 2-fumerase, (Frds(V)) designates strain over-producing fumarate reductase, (MDH2) designates strain over-producing malate dehydrogenase, (Frds(V)-FumRs 1) designates strain over-producing fumarate reductase and fumRs-fumerase, (Frds(V)-Fum1s) designates strain over-producing fumarate reductase and fum1s-fumerase. The FumR2 point represents the citrate production rate calculated after excluding the final measurement, as the citrate production rate drastically increased for the final measurement and therefore does not represent the trend observed for the rest of the measurements.

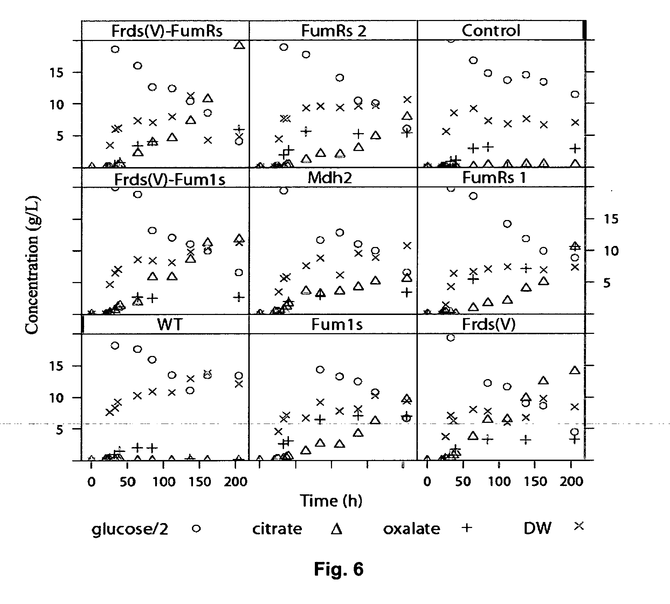

Fig. 6 shows the major fermentation products (measured in g/Litres) as well as glucose concentration (plotted as the measured value in g/0.5 litres), at timepoints 0-200 hours fermentation, were plotted for all the attempted fermentations. The strain name is printed at the top of each panel.

*A. niger* naturally produces citrate very efficiently, and it is therefore used industrially for citrate production. The present invention was made in an attempt to divert this production potential towards succinate, as succinate is of greater economic value than citrate. Currently succinate trades for approximately $4-6 per kilogram, whereas the bulk price for citrate is $0.5 per kilogram. Therefore metabolic engineering strategies involved in enabling the redirection of carbon flux towards succinate were attempted.

The strategies employed according to the present invention successfully resulted in the over-expression of genes involved in the production of malate, fumarate and succinate as well as cytosolic localisation of gene products resulting form the expression of said genes. However, the employed strategies did not result in a significant production of succinate, which was the originally aim of the experiment, but rather surprisingly resulted in a very significantly enhanced production of citrate.

Thus it was surprisingly shown that enhanced production of citrate can be achieved by over-expression of genes involved in the production of malate and/or fumarate and/or succinate. Particularly, the genes involved in the production of malate, fumarate and succinate comprise genes chosen from the group comprising malate dehydrogenases, fumarate reductases or fumarases.

Malate dehydrogenases (Mdh) reversable convert oxaloacetic acid (oxaloacetate) to malate using the cofactor NADH. Different Mdh's are localized in several compartments in the cell, such as the mitochondria and cytosol. Most fungi, such as A. *niger,* has a cytosolic and a mitochondrial Mdh activity. Preferably the malate dehydrogenase according to the invention is a cytosolic malate dehydrogenase.

However, an alternative embodiment according to the invention comprises the relocation of malate dehydrogenases from other cell compartements to the cytosol. In yeast there are three forms of malate dehydrogenase, a cytosolic, mitochondrial and peroxisomal. All of these could be used to increase cytosolic malate concentrations. Further, overproduction of malate may be achieved by overexpression of malate synthase, which lead to the increase of cytosolic malate concentration by converting glyoxylate to malate.

In eukaryotic organisms fumarases reversibly convert fumaric acid to L-malic acid in the cytosol as well as performing the same function in the tricarboxylic acid cycle (TCA cycle) in the mitochondria. The localization of the enzyme (e.g. cytosolic or mitochondrial) is most often determined by an N-terminal mitochondrial targeting sequence and protein conformation. The affinity of fumarases for either fumarate or malate as well as the intracellular concentration of fumarate and malate, determines the direction of the reaction. The affinity of fumarases from different organisms differ, for instance the affinity of S. *cerevisiae* cytosolic fumarase preferentially converts fumarate to malate, while the fumarase from R. *oryzae* preferentially converts malate to fumarate, depending on the concentrations of the two possible substrates. A. *niger* has so far only been found to have mitochondrial fumarase activity. Preferably the fumerases according to the invention are cytosolic fumarases. In a preferred embodiment according to the invention cytosolic location is achieved by relocation of fumarases from other cell compartements (e.g. mithochondria) to the cytosol.

Fumarate reductases catalyze the reduction of fumarate to succinate. This reaction can be either cytosolicly localized (named *frds1* in *S. cerevisiae)* or in the mitochondria (named *osm1* in *S. cerevisiae).* Homologues have also been found in other organisms, such as the gene *Frdg* in *Trichoderma reesei,* which is localized in the glyoxyzomes.

Fumarate reductase can be either membrane bound or soluble (such as *frds1 and* Frdg). Succinate dehydrogenase can also have a fumarate reductase action if the reaction is reversible. A. *niger* has so far only been found to have mitochondrial fumarate reductase activity. Preferably the fumeraste reductases according to the invention are soluble cytosolic fumeraste reductases. In a preferred embodiment according to the invention cytosolic location is achieved by the relocation of fumerate reductases from other cell compartements (e.g. mithochondria or cell membranes) to the cytosol.

Another enzyme according to the invention that produces fumarate is fumarate hydratase. Fumarate hydratases are mitochondrial enzymes of the tricarboxylic acid cycle that hydrates fumarate into malate. Preferably the fumarate hydratases according to the invention are soluble cytosolic fumarate hydratases. In a preferred embodiment according to the invention cytosolic location may be achieved by the relocation of fumarate hydratase from other cell compartements (e.g. mithochondria or cell membranes) to the cytosol.

It was further shown that cytosolic localisation of gene product resulting from the expression of the genes involved in the production of fumarate and succinate resulted in enhanced production of citrate. Preferably, the genes involved in the production of fumarate and succinate comprise genes chosen from the group comprising fumarate reductases or fumarases. More particularly, the genes involved in the production of fumarate and succinate comprise genes chosen from the group consisting of *Fum1, FumR* and *Frds1.* Even more preferably, the genes involved in the production of fumarate and succinate comprise genes chosen from the group consisting of *Fum1, FumR* and *Frds1* originating from *S. cerevisiae* and *R. oryzae.*

Conventional production conditions comprise high levels of oxygen, preferably >100mbar partial pressure O₂, which may be supplied as normal air (preferably in excess of 1 WM (volume/volume/minute)). The pH conditions should preferably be low pH, such as pH below 3.0. Preferably the pH is in the interval of 1.0 - 3.0.

Any temperature at which the citrate producing organism grows can potentially be used in the method according to the invention. The optimal temperature for citrate production according to the invention is, however, in the interval of 25 - 40°C, even more preferably 30 - 35 °C.

The production of citrate according to the invention is preferably performed having the producer strain submerged in the appropriate media under rapid agitation such as -500rpm, depending on the fermentor design used and oxygen requirements of the particular strain.

Conventional production media comprise sources of carbon, nitrogen, phosphate as well as trace metals. Citrate production media may in principle include any carbon source, however, the used carbon source must lead to rapid metabolism. Most appropriate carbon sources used in high levels include sucrose, glucose, molasses, etc. These carbon sources must be supplied at rates higher than 50 g/L, and are typically supplied in a range of 60 - 240 g/L. Ammonium salts, Urea etc. may be used as nitrogen sources at low concentrations (0.4-0.6 gN/L). Consumption of the nitrogen source should lead to a decrease in pH to around 2.0 - 3.0. Potassium phosphate may be used as phosphate source at low levels. Preferably the phosphate levels are suboptimal for growth and are in the range of 1 - 3.0, more preferably 0.4 - 1 g/L Phosphate. Trace metals including zink and iron may also be included at low levels of around 0.00025 zink (0.2-1.5 mg/L) and 0.0013 iron (1.3-1.5 mg/L).

The preferred organisms for use according to the invention are fungi, including yeasts. Yeasts that may preferably be used according to the invention include but are not limited to *Saccharomyces* and *Candida* species, particularly *Saccharomyces cerevisiae* and *Candida lipolytica.*

However, the preferred industrially relevant microorganisms according to the invention are filamentous fungi. Filamentous fungal strains include, but are not limited to, species of *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium,* and *Trichoderma.* Filamentous fungal strains that may preferably be used according to the invention include but are not limited to *Saccharomyces* and *Candida* species, particularly *Saccharomyces cerevisiae* and *Candida lipolytica.*

Preferably the microorganisms used according to the invention are Aspergillus sp. Among preferred aspergilli are *Aspergillus niger, Aspergillus oryzae* and *Aspergillus flavus* which, although not currently used commercially, is known for its organic acid production ability. Further useful according to the invention is *Aspergillus terreus,* as it is used for industrial itaconic acid production. However, the most preferred microorganisms for use according to the invention is *Aspergillus niger.*

In one embodiment the genes involved in the production of fumarate and succinate are truncated in order to ensure cytosolic localisation of the corresponding gene products. Particularly the genes *Fum1* and *FumR* are truncated.

According to one aspect of the invention the truncated version of *Fum1* and *FumR* (referred to as *Fum1s* and *FumRs)* are designed to lack the initial 17 and 15 amino acids respectively, which, when removed in S. *cerevisae* and R. *oryzae,* prevent the mitochondrial import of Fum1p and FumRp. It is assumed that these truncations result in purely cytosolic activity for the gene products.

The expression of a truncated form of S. *cerevisiae fum1,* which in S. *cerevisiae* is known to result in an increase in cytosolic fumarase activity (Peleg *et al.,* 1990), surprisingly lead to drastically increased citrate productivities in *A. niger* during fermentation. Accordingly there is a very strong correlation between the citrate production rate and the fumarate consumption rate towards the later stages of the fermentation, and the rate of fumarate consumption does not contribute to the production rate of citrate directly. It does however show the dependence of the fumarase and fumarate reductase genes on fumarate, indicating that it is indeed the substrate for both these enzymes.

Increased citrate productivity, similar to the productivity obtained for *fum1s* expression, may be achieved through the expression of a truncated, cytosolic targeted fumerase. Preferably the *Rhyzopus oryzae* fumarase *(fumR)* (Friedberg, Peleg, Monsonego, Maissi, Battat, Rokem, and Goldberg, Gene, 163, 139, 22-9-1995) may be used. In R. *oryzae* the overexpresion of *fumR* results in increased fumarate productivity. Surprisingly this is not the case in the filamentous fungi used according to the invention as over-expression of this fumerase surprisingly in *A. niger* leads to drastically increased citrate productivities during fermentation.

*Frds1* has previously been demonstrated to irreversibly convert cytosolic fumarate to succinate, using FADH₂ as co-factor. The expression of *Frds1* in A. *niger was* therefore expected to increase the cytosolic succinate concentration. However, expression of this fumerate reductase was surprisingly observed to lead to an increase in the citrate productivity, which was even higher than what was seen for the fumarase expressing strains. Thus, one aspect of the invention is the over-expression of fumarate reductase. Preferably the fumarate reductase is *Frds1.* Without wishing to be limited by theory it appears that fumarate is exported from the mitochondria to provide the substrate for the cytosolic fumarate reductase, and that the formed succinate then serves as substrate to the mitochondrial citrate antiporter.

To test if *fum1* or *fumR* and *frds1* expression in the same strain could have additive beneficial effects, the best citrate producing *frds1* over-expressing strain, frdsV, was transformed with *fum1s* and *fumRs.* As seen in Fig. 2 it is clear that the combination of *fumRs* and *Frds1* leads to a further increased citrate production in the later stages of the fermentation, when compared to either of the single gene expression mutants. Without wishing to be bound by theory it is believed that the co-expression of *fumR* and *Frds1* forms a cytosolic pathway from malate towards succinate, resulting in a higher concentration of succinate in the cytosol. The succinate can then be used by the citrate antiporter for enhancing the citrate production. This may, however, not be true when *fum1s* is combined with *Frds1.*

The citrate yield on glucose was also significantly increased in the double expression mutant, to an impressive 0.9 g citrate per g glucose in Frds(V)-FumRs1 during the later stages of the fermentation.

The direct correlation between the glycolytic flux and citrate production rate was demonstrated by the strong exponential correlation (R² = 0.92) between citrate production rate and glucose uptake rate between the transformants.

However, Frds(V)FumRs 1 does not correspond to this trend, as it has a higher citrate production rate than the trend suggest should be observed for its glucose consumption rate. This suggests that the dual insertion of *Frds1* and *fumRs* fundamentally improves the yield on citrate, which can clearly be observed in Fig. 3 (90% yield).

Thus, according to one embodiment of the invention the enhanced production of citrate is achieved by simultaneous over-expression of genes resulting in the production of fumarate and succinate. More particularly in one embodiment according to the invention the enhanced production of citrate is achieved by simultaneous over-expression of *fumR* and *frds1.* In another embodiment according to the invention the enhanced production of citrate is achieved by simultaneous over-expression of *fum1* and *frds1.*

Metallic ions are of great importance to citrate production. It has long been known that the presence of even trace amounts of Mn²⁺ (more than 1 ppb, Mattey M., Crit Rev.Biotechnol., 12, 87, 1992) will drastically decrease citrate production in submerged cultivation. Mn²⁺ has been reported to have multiple effects on A. *niger* physiology, e.g. morphology changes from pellet to filamentous, increased protein turnover, impaired DNA synthesis and altered composition of plasma membrane and cell walls (Karaffa and Kubicek, 2003). Traditionally, the feedstock has been de-ionised using ionexchange pre-treatment, which adds to the labour and costs involved in using substrates such as molasses. Classical selection techniques have therefore been used to select for mutants less sensitive to Mn²⁺. Normally, however, media for the conventional industrial citrate production are always treated in order to remove Mn²⁺. Conventionally, it is an aim to reach Mn²⁺ concentrations as low as 2 µg/l cultivation media or even 1 µg/l cultivation media.

The enhanced production of citrate according to the invention is particularly surprising considering the presence of trace amounts of Mn²⁺ in the production media (introduced through glucose, which was not treated with an anion adsorption resin as is normally done for citrate production). By using the method according to the invention, citrate was produced in media that were not treated in order to reduce the Mn²⁺ content, thus having a Mn²⁺ concentration at around 4µg/l. Thus, an additional benefit achieved according to the present invention seems to be that the need for this expensive and unpractical treatment to reduce Mn²⁺ may be reduced substantially.

In a preferred embodiment according to the invention the production media comprise at least 2µg/l Mn²⁺. In a more preferred embodiment according to the invention the production media comprise at least 3µg/l Mn²⁺. In an even more preferred embodiment according to the invention the production media comprise at least 4µg/l Mn²⁺.

Accordingly, the invention further relates to methods for the enhanced production of citrate, wherein the need for removal of trace Mn²⁺ is reduced.

The method according to the invention is thus also a method of improving Mn²⁺-tolerance in fungi. Even further the method according to this aspect of the invention is a method of improving Mn²⁺-tolerance in filamentous fungi. Even further the method according to this aspect of the invention is a method of improving Mn²⁺-tolerance in *Aspergillus sp.* Even further the method according to this aspect of the invention is a method of improving Mn²⁺-tolerance in *Aspergillus niger.* Particularly, the method according to this aspect of the invention is a method of improving Mn²⁺-tolerance in citrate producing fungi.

In another embodiment of the invention it was surprisingly demonstrated that the over-expression of genes involved in the production of malate also resulted in enhanced production of citrate. Though the increase in citrate production was significant according to this aspect of the invention it was, however, of a more transient nature and did not result in the production rates observed by increasing the production of fumarate and/or succinate.

Accordingly it was shown that over-expression of *Mdh2* involved in the production of malate led to an increase in the initial citrate production rate compared with all the other tested transformants. The *Mdh2* expressing strain, however, had a slower overall citrate production rate compared to the other mutants in the study.

### EXAMPLES

*Fungal strains.* All *Aspergillus niger* strains used in this study were generated from the A742 (cspA1 pyrA5) reference strain.

*Plasmids.* Manipulation of plasmid DNA and transformation of plasmids into *Escherichia coli* DH5α were carried out according to standard procedures (Sambrook and Russell, Cold Spring Harbor Laboratory Press, Cold Spring Harbor, NY, 2001). All plasmids were constructed as random genomic integrating vectors. Three of the genes *(Fum1, FumR,* and *Mdh2)* used in this study were over-expressed by the introduction of these genes into the pAT-1 vector (see Table 2).

*MDH2, Fum1s* and *FumRs* were separately introduced into plasmid pAT-1 by ligating, respectively, a 1.3kb, 1.4kb and 2 kb PCR product of *MDH2* from S. *cerevisiae* (using primer pair: Mdh2_F and Mdh2_R, see primers in table 1), a truncated *Fum1* from S. *cerevisiae* (using primer pair: Fum1s_F and Fum1s_R) and a truncated *FumR* from R. *oryzae,* (using primer pair: FumRs_F and FumRs_R) into pAT-1. The truncated version of *fum1* and *fumR* will be referred to as *Fum1s* and *FumRs* in this worK The PCR primers were designed to contain a Bsal cut site on the 3' primers and a Notl cut site on the 5' primers. The PCR products were then digested with the offset cutting BSAI (designed to result in a 5-prime overhang matching to the 3-prime overhang of Ncol in pAT-1) and Notl, into pAT-1 cut with Ncol and Notl. The truncated version of *Fum1* and *FumR* (referred to as *Fum1s* and *FumRs)* were designed to lack the initial 17 and 15 amino acids respectively. The designation of the different plasmids used in this study can be seen in Table 2.

The final gene used in this study, *frds1,* was placed on a plasmid through several steps:
1. BamHl-Hindlll fragment, containing the terminator region from *A.nidulans trpC* gene, was excised from pAN7-1, and inserted into BamHl and Hindlll sites of pUC57 (Fermentas), to construct plasmid pUC57Ter-trpC.
2. Promoter region of the A. *nidulans gpdA* gene from plasmid pAN7-1 was amplified by PCR with primers PAN1 (5'-gtg-agt-gga-tcc-ata-tgg-atg-tct-gct-caa-gcg -3', containing Ndel and BamHl sites) and M13/pUC reverse sequencing primer (Fermentas). The resulting DNA-fragment was digested with EcoRI and BamHI and cloned into EcoRI and BamHI sites of pUC57TtrpC to create the plasmid pAN-N.
3. Ndel site was disrupted in ppyrG (Oakley *et al.,* 1987) by step by step digestion with Ndel, treatment with Klenow fragment DNA polymerase I and dNTP and ligation by T4 DNA ligase. The resulting plasmid was designated as ppyrG-N.
4. Sspl-EcoRl fragment, contained *pyrG* and disrupted Ndel site, was excised from ppyrG-N and inserted into Sspl and EcoRl sites to construct plasmid pUC57Ter-trpC-pyrG-N.
5. pUC57Ter-trpC-pyrGN was consequently treated with Xbal and T4 DNA ligase to delete Xbal-Xbal fragment. The resulting plasmid was step by step treated with BamHl; Klenow fragment DNA polymerase I and dNTP; and T4 DNA ligase to disrupt BamHl site in pyrG.
6. EcoRl-Xbal fragment, containing promoter region of the A. *nidulans gpdA* and terminator region from *A.nidulans trpC,* was excised from pAN-N and cloned into pTer-trpC-pyrG-NB-Xba to create pANNPYRG.
   Yeast *frds1* was amplified by PCR with primers: frds13 (5'-aat-tgc-ata-tgt-ctc-tct-ctc-ccg-3, containing Ndel site) and frds12 (5'-tat-agg-atc-cgt-tac-ttg-cgg-tca-3', containing BamHl site). The resulting DNA-fragment was digested with Ndel and BamHl and cloned into Ndel and BamHl sites of pANNPYRG to create the plasmid pFRDS-A1.

**Table 1. Sequences of primers used to create pFum1s, pFumRs and pMDH2**

| **Primer name** | **Sequence** |
|---|---|
| Fum1s_F | gtctaGGTCTCCCATGAACTCCTCGTTCAGAACTGAAAC |
| Fum1s_R | GCGGCCGC-TTATTTAGGACCTAGCATGTGTTCAGG |
| FumRs_F | gtctaGGTCTCCCATGAACAACTCTCCTCGTCTTTTCAG |
| FumRs_R | GCGGCCGCTTTAATCCTTGGCAGAGATCATATCTTC |
| Mdh2_F | gtctaGGTCTCCCATGATTCTCCTTCTGATTCTTTTTCCC |
| Mdh2_R | TAGCGGCCGCTTAAGATGATGCAGATCTCGATGC |

**Table 2. Main plasmids used in this study**

| **Plasmid name** | **Created** | **Description** |
|---|---|---|
| pFum1_s | This study | Contains *S. cerevisiae Fum1s* expression cassette |
| pFumR_s | This study | Contains *R. oryzae FumRs* expression cassette |
| pFrds1_A1 | This study | Contains *S. cerevisiae Frds1* expression cassette |
| pMdh2 | This study | Contains S. *cerevisiae Mdh2* expression cassette |
| pAN7-1 | ^{*1} | Contains E. *coli hph* gene, confers hygromycin resistance. |
| pAT-1 | ^{*2} | Contains strong constitutive fungal gpdA promoter and trpC terminator. |
| pGemT-*pyr4* | | Contains *N. crassa pyr4* gene, cures a *pyrG* mutation (piramidine auxotrophy) in *A. niger.* |

| | | |
|---|---|---|
| ¹ (Punt, Oliver, Dingemanse, Pouwels, and van den Hondel, Gene, 56, 117, 1987) ² Tolsters A., Techinical University of Denmark, 2003 | | |

*Fungal Transformation.* PyrG- mutant A742 was transformed with plasmid pFRDS A1 containing the Frds1 gene and A. niger pyrG as marker to create strain Frds(V). These strains were then co-transformed with pFum1_s or pFumR_s, and pAN7-1 to create strains Frds(V)-Fum1s and Frds(V)-FumRs. A control strain was created where N402 was transformed with pAN7-1 alone, it will be referred to as Control in the text.

*PyrG-* mutant A742, which has the same strain background as wild type *A. niger* N402, were co-transformed with pFum1s, or pFumRs, or pMdh2 and plasmid pGemT-pyr4 containing *N. crassa pyr4* as a marker gene. The co-transformation ratio was 10:1 to the marker plasmid. Transformation of A. *niger* was achieved as described by Nielsen, Albertsen, Lettier, Nielsen and Mortensen, *Fungal genetics and biology,* 2005 for *A. nidulans.* 11µl of plasmid was added to a 100µl volume containing at least 10⁶ protoplasts and plated in selective media. The resulting strains are designated Fum1s, FumRs or MDH2, respectively.

*Media for strain development. A. niger* strains were grown at 30°C on solid or liquid minimal media (MM) (Clutterbuck, Handbook of Genetics, Plenum Press, New York, NY, 1974, 447) or MM containing uracil and uridine for *pyr⁻* auxothrophic strains. The selection of transformants was performed either on MM media or MM containing 200-500 µg/ml hygromycin B. Bacterial strains were grown at 37°C in LB medium with 100 µg ampicillin/mL.

*Inoculum.* The strains were stored at -80°C in eppendorf tubes containing 0.01 % Tween with 20% (v/v) glycerol. From the stock tubes, point inoculations with a toothpick on plates with selective media for plasmid bearing strains were used.

*Cultures grown in shake flasks.* Baffled, cotton-stopped, 500 mL Erlenmeyer flasks were used. These flasks contained a volume of 100 mL medium with the following compositions: MM media when protoplasts for fungal transformations were to be obtained, or Kristiansen media (Kristiansen B. and Sinclair C.G., Biotechnology and Bioengineering, XXI, 297, 1979) for screening of transformants. The pH was adjusted to 5.2 with NaOH and autoclaved along with the glucose solution. The shake flasks were grown at 30°C and 150 rpm for up to 2 weeks.

*Medium for the batch cultivations.* The medium for the batch cultivations had the following composition: 50g/L NH₄NO₃; 50g/L KH₂PO₄; 10 g/L MgSO₄·7H₂O; 1 mUL 1000 X trace element solution containing per litre: 0.037g/L FeCl₃, 0.06g/L CuSO₄.5H₂O, 0.1g/L ZnSO₄.7H₂O; 50 µL/L antifoam (Sigma, A-8436); and 50 g/L glucose (Kristiansen B. and Sinclair C.G., Biotechnology and Bioengineering, XXI, 297, 1979).

*Cultivation conditions.* The cells were grown as aerobic batch cultivations with nitrogen eventually being the growth-limiting component. The cultivations were carried out in well-controlled 5-litre in-house-manufactured bioreactors with working volumes of 4 litres. The bioreactors were equipped with two disk turbine impellers rotating at 500 rpm. The pH was initially set to 5, 150 rpm agitation, and with an airflow of 0.1 VVM. After 16 hours the airflow was then increased to 4 litre per min. (1 VVM), and the agitation set to 500rpm. The pH was allowed to decrease without further control. The off gas passed through a cooled condenser to limit evaporation from the bioreactor.

*Cell mass determination.* The cell mass concentration on a dry weight basis was determined by the use of nitrocellulose filters with a pore size of 0.45 µm (Gelman Sciences, Ann Arbor, Mich.). Initially, the filters were pre-dried in a microwave oven at 150 W for 10 min., and then weighed. A known weight of cell culture was filtered, and the residue was washed with distilled water. Finally, the filter was dried in the microwave at 150 W for 15 min., and then weighed.

*Analysis of extracellular metabolites.* For the determination of the extracellular metabolites, a sample was taken out of the bioreactor and immediately filtered through a 0.45 µm-pore-size cellulose acetate filter (Osmonics, Minnetonka, MN). The filtrate was frozen and kept at -20°C until analysis. Organic acids and glucose were separated on an Aminex HPX-87H column (Biorad, Hercules, Calif.) at 65°C, using 5 mM H₂SO₄ as the mobile phase at a flow rate of 0.6 mL/min and detected refractometrically (Waters 410 Differential Refractometer Detector; Millipore Corp., Milford, Mass.).

### Example 1. The effect of cytosolic fumarase over-expression on citrate production.

*A. niger* strains overproducing fumerase were assayed for citrate production. The results are shown in Fig. 1. Significantly increased citrate productivities and yields were observed when two different cytosolic targeted fumarases, *fum1* and *fumR* (from S. *cerevisiae* and R. *oryzae,* respectively), were expressed. The strains were compared under several different conditions, including shake flask cultures and different batch cultivation conditions (results not shown). In all instances higher yields and productivities were observed for the strains containing *fum1s* or *fumRs* compared with the wild type. From Figs. 1 and 2 it can be seen that the wild type and control strains do not produce citrate in the batch fermentations, however, the wild type did produce citrate in shake flask culture, but still at a slower rate than the transformants.

### Example 2. The effect of the insertion of Frds1 and MDH2 as well as the effect of the secondary insertion of fumRs or fum1s into the frds1 containing Frds(V) strain.

In Fig. 2 the effect of expressing S. *cerevisiae Frds1* in *A. niger* can be seen. Once again higher citrate yields and productivities were obtained. Indeed, the *frds1* containing transformants had the highest productivities of all the strains tested. The insertion of *fumRs,* but not *fum1s,* into the Frds(V) strain background, however, resulted in a further increased citrate production compared to the single gene insertion *frds1* (Frds(V)) strain, particular in the later stages of the fermentation.

### Example 3. Over-expression of malate dehydrogenase in A. niger

Expression of the *MDH2* gene from *S. cerevisiae* in A. *niger* also resulted in an increased citrate production compared with the wild type and control. When compared with the other transformants, it also results in an increase in the initial citrate production rate. However, except for the initial stages of the fermentation, it performs poorly compared to the other gene insertion transformants.

### Example 4. Comparison of citrate yield for all experimental strains.

All constructed strains were tested for their ability to produce citrate. The results are shown in Fig. 3. The yield of citrate on glucose, which is another important parameter for optimization of citrate production, is most significantly increased by the insertion of either *Fum1s* or *FrdsV,* or the dual insertion of *Frds1* and *FumRs* (see Fig. 4). A direct correlation between the glucose consumption rate and the citrate production rate was also found for the transformants, except for FrdsV-FumRs 1, which had a significantly higher citrate production rate compared to its glucose consumption rate. A very strong Pearsons correlation (higher than 0.98) for all the transformants was found between citrate and pyruvate production. A strong correlation between the fumarate consumption rate and the citrate production rate was also observed for all the transformants containing either a fumarase or fumarate reductase gene (see Fig. 5).

## Claims

1. A method for enhanced fungal production of citrate comprising the steps of:
i. providing a fungus capable of producing citrate in a conventional production media,
ii. providing conventional conditions for fungal production of citrate,
iii. providing cytosolic over-production of malate and/or fumarate and/or succinate in said fungal organism.

2. A method according to claim 1, wherein the cytosolic over-production of malate and/or fumarate and/or succinate in said microbial organism is provided by cytosolic over-expression of genes and/or cytosolic localization or gene products responsible for production of malate and/or fumarate and/or succinate.

3. A method according to claim 2, wherein genes or gene products responsible for production of malate and/or fumarate and/or succinate comprise genes chosen from the group consisting of genes encoding malate dehydrogenases, malate synthases, fumarate reductases, fumarate hydratases or fumarases or their corresponding gene products.

4. A method according to claim 3, wherein genes or gene products responsible for the production of malate and/or fumarate and/or succinate comprise genes chosen from the group consisting of genes encoding malate dehydrogenases, fumarate reductases or fumarases or their corresponding gene products

5. A method according to claim 4, wherein genes or gene products responsible for the production of malate and/or fumarate and/or succinate comprise genes chosen from the group consisting of *MDH2, Fum1, FumR* and *Frds1* or their corresponding gene products.

6. A method according to claim 5, wherein genes or gene products responsible for the production of fumarate and/or succinate comprise genes chosen from the group consisting of *Fum1, FumR* and *Frds1* or their corresponding gene products.

7. A method according to any of the above claims comprising simultaneous cytosolic over-expression of *FumR* and *Frds1* and/or simultaneous cytosolic localisation of their corresponding gene products.

8. A method according to any of the above claims 1 -6 comprising simultaneous cytosolic over-expression of *Fum1* and *Frds1* and/or simultaneous cytosolic localisation of their corresponding gene products

9. A method according to any of the above claims, wherein the genes or gene products have been truncated in order to remove the amino-terminal mitochondria-directing signalling sequences.

10. A method according to any of the above claims, wherein the fungal organism is a filamentous fungus.

11. A method according to claim 10, wherein the filamentous fungus is an aspergillus sp.

12. A method according to claim 11, wherein the microbial organism is *Aspergillus niger.*

13. A method according to any of the above claims, wherein the production media is a media for submerged production.

14. A method according to claim 13, wherein the production media or its constituents have not been treated to reduce the content of Mn²⁺.

15. A method according to claim 13 or 14, wherein the production media has a Mn²⁺ content of above 1 µg/l, preferably above 2µg/l even more preferably above 4µg/l.

16. A method for improving the Mn2+ tolerance in a fungal organic acid producer species comprising the steps of:
i. providing a fungus capable of producing said organic acid in a conventional production media,
ii. providing conventional conditions for fungal production of said organic acid,
iii. providing cytosolic over-production according to any of claims 1-9 of malate and/or fumarate and/or succinate in said fungal organism.
